# EUROPÄISCHE PATENTSCHRIFT

(11) **EP 0 401 347 B1**
(45) Veröffentlichungstag und Bekanntmachung des Hinweises auf die Patenterteilung: **20.03.1996**
(21) Anmeldenummer: 90900758.5
(22) Anmeldetag: 14.12.1989
(51) Int. Cl.: A61K 38/16

(54) **VERWENDUNG VON TRANSFERRIN ZUR BEKÄMPFUNG DER TOXISCHEN WIRKUNG DES ENDOTOXINS BEI DER ENDOTOXINÄMIE**
USE OF TRANSFERRIN TO COMBAT THE TOXIC EFFECTS OF ENDOTOXINS IN ENDOTOXEMIA
UTILISATION DE LA TRANSFERRINE POUR COMBATTRE LES EFFETS TOXIQUES DE L'ENDOTOXINE EN CAS D'ENDOTOXEMIE

(30) Priorität: 15.12.1988 DE 3842143
(43) Veröffentlichungstag der Anmeldung: 12.12.1990
(73) Patentinhaber: NITSCHE, Dietrich, D-24105 Kiel (DE)
(72) Erfinder: NITSCHE, Dietrich, D-24105 Kiel (DE)
(74) Vertreter: Tönnies, Jan G., Dipl.-Ing.
(86) Internationale Anmeldenummer: DE8900773
(87) Internationale Veröffentlichungsnummer: WO9006768

(56) Entgegenhaltungen:
- Arzneimittel-Forschung/Drug Research, Band 38(I), Nr. 6, 1988; D. BERGER et al.: "Comparison of the Endotoxin-Binding Capacity of Human Transferrin and a Human Applicable Immunoglobulin Preparation", seiten 817-820
- Progess in Clinical and Biological Research, Band 272, Bacterial Endotoxins: Pathophysiological Effects, Clinical Significance, and Pharmacological Control, 1988, Alan R. Liss, Inc.; D. BERGER et al.: "Quantification of the Endotoxin-binding capacity of human transerrin", pages 115-124
- CHEMICAL ABSTRACTS, Band 109, Nr. 16, 17. Oktober 1988, Columbus, Ohio, US, siehe Seite 378
- CHEMICAL ABSTRACTS, Band 109, Nr. 24, 12. Dezember 1988, Columbus, Ohio, US, siehe Seiten 325-326
- CHEMICAL ABSTRACTS, Band 111, Nr. 20, 13. November 1989, Columbus, Ohio, US, siehe Seite 428
- CHEMICAL ABSTRACTS, Band 110, Nr. 12, 20. Marz 1989, Columbus, Ohio, US, siehe Seite 430

## Beschreibung

Die Erfindung betrifft die Verwendung eines Mittels bestehend aus Transferrin zur Herstellung eines Arzneimittels zur Bekämpfung der toxischen Wirkung des Endotoxins bei Endotoxinämien, dadurch gekennzeichnet, daß das Transferrin Eisen gebunden hat.

Transferrin ist ein Glykoprotein mit einem durchschnittlichen Molekulargewicht von 80 000 D. Es ist im menschlichen und tierischen Plasma in einer Konzentration von ca. 290 mg/dl ( Normalbereich beim Menschen: 400 mg/dl - 200 mg/dl) vorhanden. Bisher wird als Hauptfunktion des Transferrins seine Fähigkeit angesehen, spezifisch dreiwertiges Eisen zu binden und zu transportieren, wobei es ein oder zwei Moleküle Eisen binden kann. Auf diese Weise wird das Eisen nach seiner Resorption im Dünndarm zu den Eisendepots in der Leber, in der Milz und zu den Retikulozyten im blutbildenden Gewebe transportiert.
Als eine weitere wichtige Funktion des Transferrins wird seine bakteriostatische Eigenschaft angesehen (Martin CM, Jandl JH, Finland M ; J.Infect.Dis.(1963) 112: 158 -163; Fletcher J: Immunology(1971) 20: 493 - 500 ). Eisen ist ein wesentlicher Wachstumsfaktor für die Bakterien. Durch die Komplexbildung des Eisens mit dem Transferrin wird die Konzentration an freiem Eisen im Plasma unter dem Wert gehalten, der für das Wachstum der Bakterien erforderlich ist. Aus dem bakteriostatischen Effekt des Transferrins wird von Berger und Beger ( Berger und Beger: Arzneim.-Forsch./Drug Res.(1988) 38: S. 817 - 820: und Prog. Clin. Biol.Res. (1988) 272: S. 115 - 124 ) geschlossen, daß dem Transferrin, in der eisenfreien Form, bei ausgedehnten gramnegativen Infektionen sogar eine gewisse Bedeutung als Zusatztherapeutikum für die antimikrobielle Therapie als Ergänzung zu der herkömmlichen Antibiotikatherapie zukommen könnte. Es bietet den Vorteil, daß aufgrund seines Wirkungsmechanismus keine Resistenzentwicklung zu erwarten ist.

Das Endotoxin ist ein Zellwandbestandteil der gramnegativen Bakterien, das erst bei dem Bakterienzerfall freigesetzt wird. Es ist ein Makromolekül mit einem Molekulargewicht bis zu 1 x 10⁶ Dalton, das sich vorwiegend aus Zuckerverbindungen und Fettsäuren zusammensetzt. Daneben kann es noch Proteinreste angelagert haben, die von der Bakterienwand stammen. Das Endotoxinmolekül ist aus drei strukturell und immunbiologisch unterschiedlichen Subregionen aufgebaut:
Die Subregion 1, die O-spezifische Kette, besteht aus mehreren, sich wiederholenden Oligosaccharideinheiten, die jeweils von maximal 5 Neutralzuckern gebildet werden. Die Anzahl der Oligosaccharide ist von der Bakterienart abhängig; z.B. hat das hier untersuchte Endotoxin von S. abortus equi in dieser Region 8 Oligosaccharid-Einheiten. Die Subregion 2, das Kernoligosaccharid besteht u.a. aus N-Acetylglucosamin, Glucose, Galactose, Heptose und 2-Keto-3-Desoxyoctonsäure.
Die Subregion 3, das Lipid-A ( MG 2000 Dalton ) besteht aus einem phosphorylierten D-Glukosamin-Disaccharid, an das mehrere - ca. 7 - langkettige Fettsäuren in Amid- und Esterbindung geknüpft sind. Der Träger der toxischen Eigenschaften ist das Lipd-A, wobei die toxische Wirkung von einigen Fettsäureresten in dieser Region ausgeht.

Aufgrund der Größe des Endotoxin-Moleküls und aufgrund seiner Ladungsverhältnisse können sich verschiedene Verbindungen und Proteine an den vielen Gruppen bzw. Seitenketten der drei verschiedenen Subregionen des Endotoxins anlagern, ohne dadurch irgendeine Beeinflussung der toxischen Eigenschaften des Endotoxins herbeizuführen. Physiologischerweise ist an das Lipid-A ein Protein gebunden, das sog. Lipid-A-assoziierte Protein. Die Abtrennung dieses Proteinanteils von dem Endotoxin führt bei einigen Endotoxinen zu keinerlei Veränderung der toxischen Wirkung. Bei vielen Endotoxinen wurde jedoch gefunden, daß die Bindung von Proteinen an das Endotoxin eine wesentliche Steigerung der toxischen Wirkung zur Folge haben kann ( Rietschel E.TH. et al.(1982) : " Bacterial Endotoxins: Chemical Structure, Biological Activity and Role in Septicaemia " Scand. J. Infect. Dis. Suppl. 31: 8 - 21; S. 17; ). Beispielsweise wurde beobachtet, daß bestimmte Endotoxine mit einem angelagerten Proteinanteil ca 100-fach aktiver sein können, als die Endotoxine, von denen der Proteinanteil abgetrennt wurde ( Morrison DC, Oudes ZG, Betz J (1980): The role of lipid A and lipid A-associated protein in cell degranulation mechanisms. In: Eaker D. Wadstrom T (Eds). NATURAL TOXINS, pp 287 -294, Pergamon Press. New York ). Bei tierexperimentellen Untersuchungen wurde ebenfalls eine Abnahme der toxischen Wirkung der den Tieren applizierten Endotoxine beobachtet, wenn deren Proteinanteil abgespalten war ( Hitchcock PJ and Morrison DC (1984) "The protein component of bacterial endotoxins". In: E.T. Rietschel (editor) HANDBOOK of ENDOTOXINS, Vol 1: Chemistry of Endotoxin, pp 339-375, Elsevier Science Publishers B.V. ).

Berger und Beger ( Arzneim.-Forsch./Drug Res.(1988) 38: S. 817 - 820: und Prog. Clin. Biol.Res. (1988) 272: S. 115 - 124 ) haben durch Isotopenuntersuchungen gezeigt, daß sich auch menschliches Transferrin, und zwar nur in der eisenfreien Form, an Endotoxin binden kann. Weiterhin wurde eine Anlagerung von α₂-Makroglobulin sowie von Gc-Globulin an Endotoxin nachgewiesen. Aufgrund dieser Untersuchungsergebnisse wurde von den Autoren der Druckschriften postuliert, daß dem Transferrin in seiner eisenfreien Form möglicherweise eine physiologische Bedeutung als " Carrier- Protein" für Endotoxin im Organismus zukommt. Eine ähnliche " Carrier-Funktion" wird den High-Density-Lipoproteinen zugeschrieben (Munford RS, Hall CL, Dietschy JM (1981): " Binding of salmonella typhimurium lipopolysaccharides to rat high-density lipoproteins" Infect.Immun. 34: 835 - 843). Aus dem mit Isotopen geführten Nachweis der Anlagerung von eisenfreiem Transferrin an das Makromolekül Endotoxin ergeben sich für den Fachmann jedoch keinerlei Hinweise bzw. Rückschlüsse darauf, ob und in welchem Ausmaß durch diese Anlagerung auch eine Beeinflussung der biologischen Aktivität. d.h. die toxischen Wirkung des Endotoxins zu erwarten ist.

In einer vergleichenden Untersuchung wurde von Berger u. Beger ( Arzneim. -Forsch./Drug Res.(1988) 38: S. 817 - 820: und Prog. Clin. Biol.Res. (1988) 272: S. 115 - 124 ) durch Isotopenuntersuchungen auch geprüft, ob eine IgG-Präparation, die zur Behandlung von primären und sekundären Immundefekten eingesetzt wird, in der Lage ist, Endotoxin zu binden ( Arzneim.- Forsch./Drug Res.(1988) 38: S. 817 - 820;). Eine Bindung des Endotoxins an das IgG konnte von den Autoren nicht nachgewiesen werden. Der fehlende Nachweis einer Bindung von Endotoxin durch die vergleichend untersuchte IgG-Präparation legt damit nahe, daß Immunglobulinen im Organismus als Träger von Endotoxin keinerlei Bedeutung zukommt. Eine Verbesserung der " Carrier-Funktion " durch die Kombination von Transferrin und IgG ist aufgrund dieser Untersuchungen nicht zu erwarten. Hinweise für eine Beeinflussung der biologischen Aktivität der Endotoxine durch die Immunglobuline ergeben sich aus diesen Untersuchungen nicht.

Endotoxin tritt normalerweise auch beim Gesunden aus dem Darm in die Blutbahn über. Zum Schutz vor einer toxischen Organschädigung hat das Plasma des Gesunden die Fähigkeit, das ständig aus dem Darm übertretende Endotoxin zu inaktivieren. Bei einem massiven Endotoxineinstrom erschöpft sich die Fähigkeit des Plasmas zur Inaktivierung des Endotoxins rasch und es tritt vermehrt biologisch aktives Endotoxin im Plasma auf, welches dann zu dem klinischen Bild der Endotoxinämie führt.

Eine vermehrter Endotoxinübertritt in die Blutbahn ist besonders bei ausgedehnten gramnegativen Infektionen, nach Gabe eines bakteriziden Antibiotikums zu erwarten. Das Endotoxin führt dann zur Freisetzung schädigender Mediatoren sowie zur Störung des Energiestoffwechsels der Zelle, woraus ein Zelluntergang und - bei höherer Endotoxindosis - schließlich ein Organversagen resultieren kann. Das klinische Bild der Sepsis korreliert in den meisten Fällen mit dem Verlauf und der Höhe der Endotoxinkonzentration im Blut ( Nitsche et al., Intensive Care Med. 12 Suppl. (1986) 185 ). Aus diesem Grund sind bei Erkrankungen, bei denen durch einen vermehrten Endotoxinübertritt in das Blut eine Endotoxinämie auftritt, zusätzliche therapeutische Maßnahmen wünschenswert, mit denen die Endotoxinaktivität im Blut herabgesetzt werden kann, um damit den klinischen Verlauf dieses Krankheitsbildes günstig zu beeinflussen.

Wie tierexperimentelle Studien gezeigt haben, scheint eine Herabsetzung der Endotoxinaktivität im Plasma durch Hyperimmunglobulin-Präparationen, die selektiv mit Antikörpern gegen den toxischen Teil des Endotoxins angereichert sind ( Lipid-A-Antikörper ), möglich zu sein. Immunglobulin-Präparationen mit einer selektiven Anreicherung von Lipid-A-Antikörpern stehen jedoch bisher nur in geringen Mengen für therapeutische Zwecke zur Verfügung, da die Auswahl entsprechender Spender sehr aufwendig ist. Bis zu einem gewissen Grad kann die Endotoxinaktivität im Plasma auch durch die Gabe polyvalenter 7S IgG-Päparationen herabgesetzt werden, was vermutlich auf deren Anteil an Lipid-A-Antikörper zurückzuführen ist. Eine gewisse Verbesserung der Endotoxinneutralisation wird durch die Anreicherung der IgG-Präparation mit Immunglobulinen der IgM-Fraktion erreicht, in der ein höherer Lipd-A-Antikörper Titer zu erwarten ist( Appelmelk BJ et al(1987), Microbiol Pathogenesis 2: 391 -393 )

Alle bisher für die Therapie verfügbaren Möglichkeiten weisen entscheidende Nachteile auf, die darin liegen, daß sie nur einen gewissen Prozentsatz der in die Blutbahn übertretenden Endotoxine inaktivieren. Bei einem starken Endotoxineinstrom, wie er beispielsweise auch bei der diffusen Peritonitis zu erwarten ist, zirkulieren trotz dieser therapeutischen Maßnahmen noch immer genügend biologisch aktive Endotoxine in der Blutbahn, was bei längerer Dauer eine toxische Organschädigung herbeiführen kann.

Der Erfindung liegt die Aufgabe zugrunde, ein Mittel zu finden, das als Therapeutikum für die Bekämpfung der toxischen Wirkung der Endotoxine geeignet ist, wenn diese bei krankhaften Zuständen vermehrt in die Blutbahn übertreten.

Erfindungsgemäß wird diese Aufgabe gelöst durch die Verwendung von Transferrin. Dabei kann das Transferrin frei von Eisen sein oder aber Eisen gebunden haben. Als besonders wirksam hat sich eine Kombination von Transferrin mit Immunglobulinen erwiesen, die entweder nur IgG oder IgG und IgM sowie IgA enthalten. Es ist auch möglich, das Transferrin in einer Serumkonserve anzureichern, um es mit den darin vorhandenen Plasma-Proteinen zu kombinieren.

Zur Prüfung der Endotoxininaktivierung durch Transferrin sowie durch die Kombination von Transferrin mit Immunglobulinen wurden folgende Untersuchungen durchgeführt:
Transferrin in der eisenfreien Form ( sog. Apotransferrin ) und Transferrin, welches Eisen gebunden hat, wurden allein sowie in Kombination mit Immunglobulinpräparationen in vitro und in vivo untersucht, inwieweit sie in der Lage sind, die biologische Aktivität von Endotoxin herabzusetzen.

### Untersuchungen in vitro:

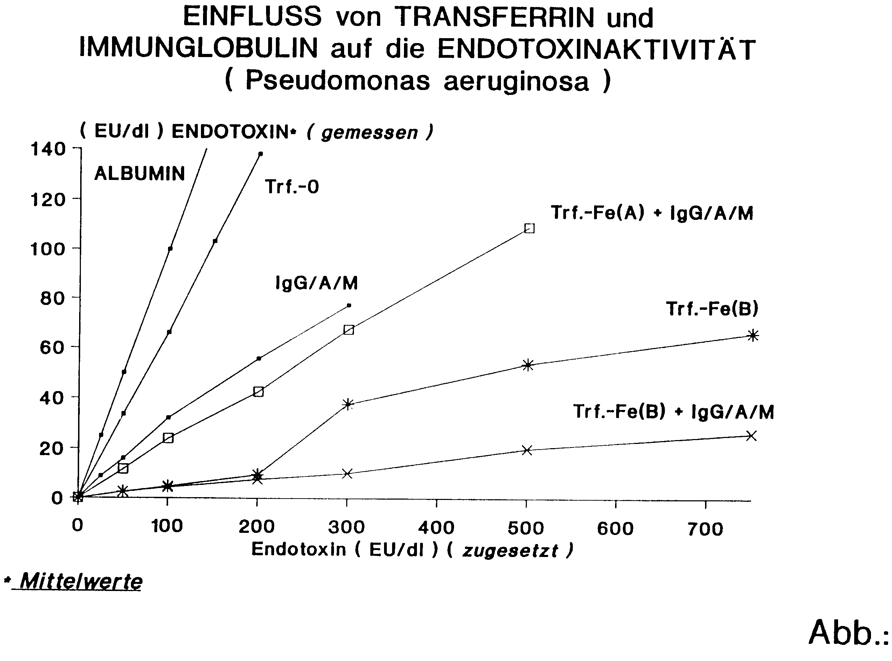

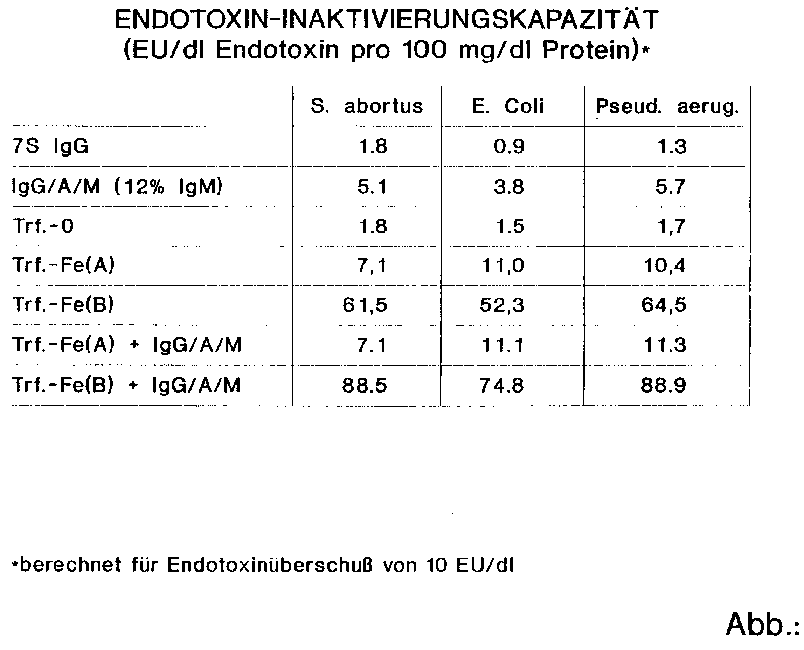

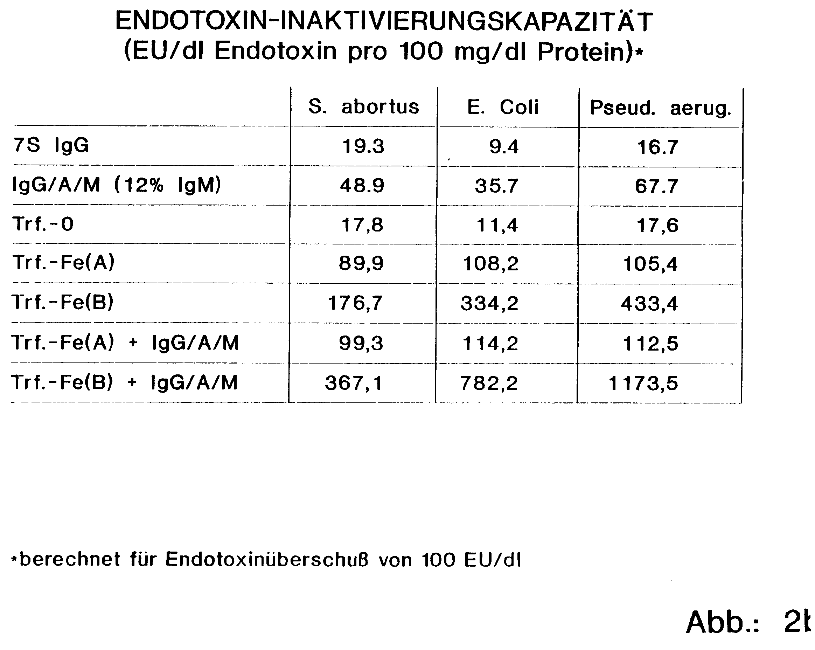

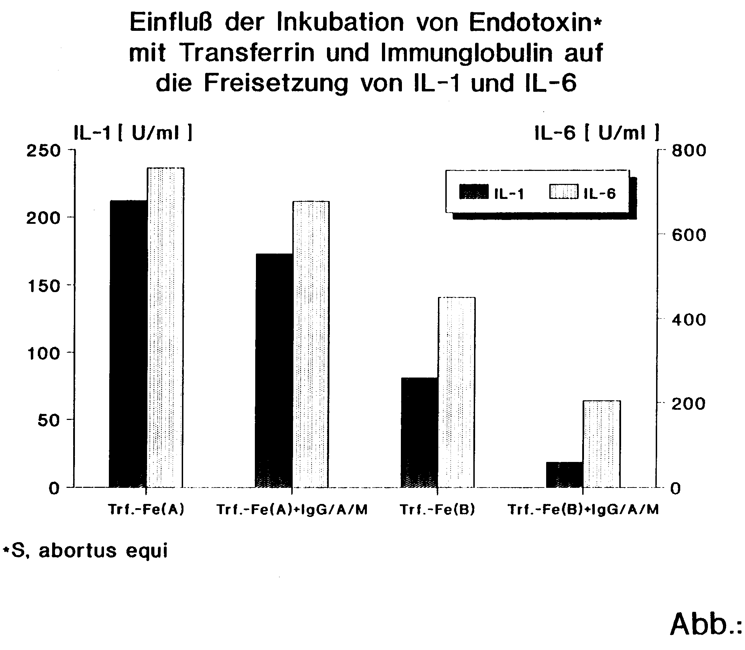

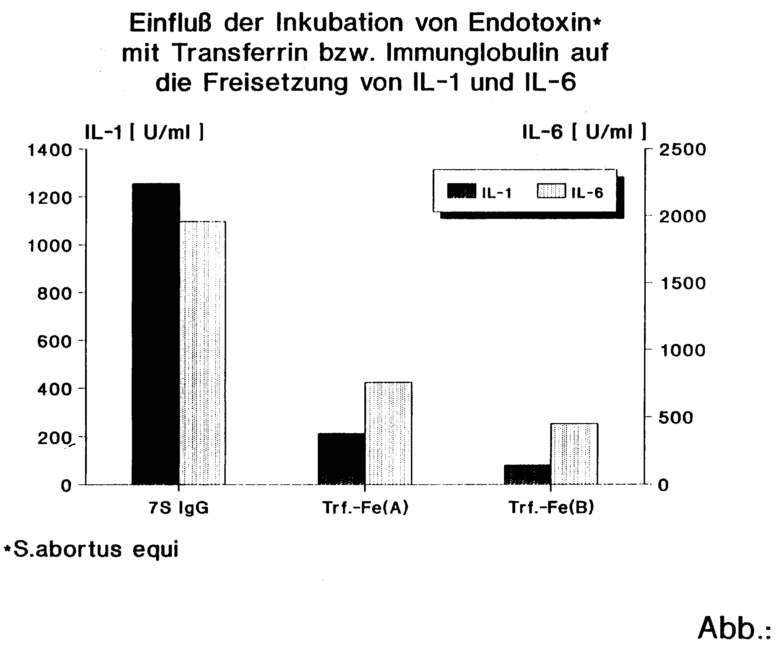

A) Quantitative Untersuchungen zum Einfluß auf die biologische Aktivität:
   Humanes Transferrin und Transferrin in Kombination mit Immunglobulin wurden mit Endotoxin in steigender Konzentration 60 Minuten bei 37 °C inkubiert. Nach Abschluß der Inkubation erfolgte die quantitative Bestimmung der biologischen Aktivität des Endotoxins im Überstand mit einer Modifikation des Limulus-Test, unter Verwendung eines chromogenen Substrates ( Nitsche et al. In: Watson, SW, Levin J, Novitsky TJ (eds) DETECTION OF BACTERIAL ENDOTOXINS WITH THE LIMULUS AMEBOCYTE LYSATE TEST. pp 417-429 ( 1987 )). Die untere Nachweisgrenze liegt bei dieser Modifikation des Limulus-Tests bei 1 EU/dl. Die mit dem Limulus-Test erfaßte Endotoxinaktivität korreliert mit der biologischen Aktivität des Endotoxins ( Nitsche D, Flad HD und Blum B (1990) Endotoxin determination by the Limulus-Test and the biological activity of endotoxin. Does a correlation exist? In Vorbereitung ).
   Für die Untersuchungen wurde Transferrin ( Trf.-Fe(A)) verwendet, welches in einer 5% Lösung einen Eisengehalt von 25 µg/dl hat, d.h. 5 µg Eisen pro Gramm Transferrin, sowie Transferrin ( Trf. -Fe(B) ) mit einem Eisengehalt von 950 µg/dl in einer 5% Lösung, d.h. 190 µg Eisen pro Gramm Transferrin.
   Außerdem wurde Transferrin verwendet, welches weitgehend frei von Eisen war ( Trf.-0̸ ). Hier konnte bei der Eisenbestimmung mit der von Megraw und Bouda ( Megraw RE, Hritz AM, Babson AL und Carroll JJ (1973) Clin. Biochem. 6: 266: Bouda J.(1968) Clin. Chem. Acta 21:159 ) beschriebenen Methode bis zu der unteren Nachweisgrenze, die bei 0.5 µg/dl liegt, in der 5% Lösung kein Eisen nachgewiesen werden.
   Folgende Immunglobuline wurden verwendet: 7 S IgG ( Intraglobin^{R}, Sandoglobin^{R}) sowie 7S-IgG, angereichert mit 12% IgM ( Pentaglobin^{R} ).
   Von folgenden Bakterienstämmen wurden die Endotoxine verwendet: Salmonella abortus equi ( NOVOPYREXAL^{R} ), Pseudomonas aeruginosa Fisher Typ 7, sowie der FDA Endotoxinstandard EC-5 von E. coli O113:H10:K0.
   BESTIMMUNG DER INAKTIVIERUNGSKAPAZITÄT FÜR ENDOTOXIN:
   Um die Fähigkeit von Transferrin sowie der Kombination von Transferrin mit Immunglobulinen zur Ianktivierung von Endotoxin zu quantifizieren, wurde die " INAKTIVIERUNGSKAPAZITÄT " bestimmt. Aus Gründen der Meßgenauigkeit wurde die Bestimmung dieser Größe nicht im Äquivalenzbereich, sondern bei einem Überschuß von 10 EU/dl und 100 EU/dl Endotoxin durchgeführt.
   Für die Berechnung dieses Parameters wurde diejenige Endotoxinmenge [EU/dl] ermittelt, die der jeweiligen Proteinpräparation zugesetzt werden muß, damit die Konzentration der freien Endotoxine im Überstand nach Inkubation (60 Minuten, 37°C ) mit der Proteinpräparation noch 10 EU/dl bzw. 100 EU/dl beträgt. Diese Endotoxinkonzentration wurde aus der Beziehung berechnet, die zwischen der zugesetzten Endotoxinmenge und der nach der Inkubation im Überstand gemessenen Endotoxinkonzentration für die jeweilige Präparation ( Transferrin, Immunglobulin und Kombination von Transferrin und Immunglobulin ) gefunden wurde. Zur Bestimmung der Inaktivierungskapazität wurde eine Meßserie durchgeführt, bei der Endotoxin in steigender Konzentration ( 10 EU/dl, 25 EU/dl, 50 EU/dl, 100 EU/dl, 200 EU/dl, 300 EU/dl, 500 EU/dl sowie 750 EU/dl und 1000 U/dl] mit den verschiedenen Protein-Präparationen 60̸ Minuten inkubiert wurde. Die Transferrin- bzw. Immunglobulinkonzentration im Reaktionsansatz betrug 312,5 mg/dl
   Bei Untersuchung der Kombination von Transferrin und Immunglobulin betrug die Konzentration jedes Proteinanteils im Reaktionsansatz 312 mg/dl. Die auf diese Weise ermittelte Endotoxinmenge stellt nach Abzug des vorgegebenen Endotoxinüberschuß von 10 EU/dl bzw. 100 EU/dl die mittlere Endotoxinaktivität[EU/dl] dar, die von der entsprechenden Proteinlösung[mg/dl] unter den jeweiligen Bedingungen inaktiviert werden kann. Bezogen auf eine Gesamtproteinkonzentration von 100 mg/dl stellt diese Endotoxinaktivität ein Maß für die " mittlere Inaktivierungskapazität [ EU/100 mg ] " der jeweiligen Transferrin-Präparation bzw. der Kombination von Transferrin mit Immunglobulin dar.
   Ergebnisse:(Abb. 1,2)
   1. Transferrin:
      Bei Inkubation von Endotoxin mit Transferrin (Trf. -0̸), welches frei von Eisen ist, findet sich, je nach Art des Endotoxins und Endotoxinkonzentration eine mittlere Abnahme der Endotoxinaktivität von 26,2% bis 36%.
      Bei Inkubation von Endotoxin mit Transferrin (Trf.-Fe(A)), welches einen Eisengehalt von 25 µg/dl - bezogen auf eine 5% Transferrin-Lösung - hat, kommt es je nach Endotoxin zu einer mittleren Abnahme der Endotoxinaktivität von 72% bis 78,7%.
      Bei einem Eisengehalt des Transferrins ( Trf.-Fe(B)) von 950 µg/dl - bezogen auf eine 5 % Transferrin-Lösung - wird eine Abnahme der Aktivität, je nach Endotoxin, von 85,3% bis 95,5% erreicht.
      a) Bezogen auf einen Endotoxinüberschuß von 10 EU/dl beträgt die Inaktivierungskapazität;
         I.) bei Trf.-0̸ für S. abortus equi 1,8 EU/100 mg, für E.Coli 1.5 EU/100 mg und für Pseudomonas aeruginosa 1.7 EU/100 mg.
         II.) bei Trf.-Fe(A) für S. abortus equi 7,1 EU/100 mg, für E.Coli 11.0 EU/100 mg und für Pseudomonas aeruginosa 10,4 EU/100 mg.
         III.) bei Trf.-Fe(B) für S. abortus equi 61,5 EU/ 100 mg, für E. Coli 52,3 EU/100 mg und für Pseudomonas aeruginosa 64,5 EU/100 mg.
      b) Bezogen auf einen Endotoxinüberschuß von 100 EU/dl beträgt die Inaktivierungskapazität:
         I.) bei Trf.-0̸ für S. abortus equi 17,8 EU/100 mg, für E.Coli 11,4 EU/100 mg und für Pseudomonas aeruginosa 17,6 EU/100 mg.
         II.) bei Trf.-Fe(A) für S. abortus equi 89.9 EU/100 mg, für E.Coli 108.2 EU/100 mg und für Pseudomonas aeruginosa 105,4 EU/100 mg.
         III.) bei Trf.-Fe(B) für S. abortus equi 176,7 EU/100 mg, für E. Coli 334,2 EU/100 mg und für Pseudomonas aeruginosa 433,4 EU/100 mg.
   2.Transferrin und 7S IgG
      Die Kombination von Transferrin mit einer 7S IgG -Präparation führt zu einer Verbesserung der Inaktivierung von Endotoxin. Bei Inkubation von Endotoxin mit Transferrin ( 312 mg/dl) und 7S IgG ( 312 mg/dl) wird die Endotoxinaktivität bei Verwendung von Transferrin (Trf.-0̸) welches frei von Eisen ist, bei Endotoxinkonzentrationen unter 200 EU/dl um 65.5 % bis 78 % herabgesetzt. Bei höheren Endotoxinkonzentrationen beträgt die Herabsetzung der Endotoxinaktivität unter diesen Bedingungen nur noch 47,1% bis 54,9%.
      Wird stattdessen Transferrin (Trf.-Fe(A)) verwendet, welches einen Eisengehalt von 25 µg/dl - bezogen auf eine 5% Transferrin-Lösung - hat, dann beträgt die Herabsetzung der Endotoxinaktivität, je nach Endotoxin 82% bis 88.8%.
      Bei einem Eisengehalt des Transferrins ( Trf.-Fe(B)) von 950 µg/dl - bezogen auf eine 5% Transferrin-Lösung - und 7S IgG, wird die Endotoxinaktivität, je nach Endotoxin, um 89,3 bis 94,2 reduziert.
      a) Wird ein Endotoxinüberschuß von 10 EU/dl zugrunde gelegt, dann beträgt die Inaktivierungskapazität:
         I.) bei Trf.-0̸ und 7S IgG für S. abortus equi 6,1 EU/ 100 mg, für E. Coli 10,6 EU/100 mg und für Pseudomonas aeruginosa 11,4 EU/100 mg.
         II.) bei Trf.-Fe(A) und 7S IgG für S. abortus equi 26.4 EU/ 100 mg, für E. Coli 24,5 EU/100 mg und für Pseudomonas aeruginosa 24,8 EU/100 mg.
         III.) bei Trf.-Fe(B) und 7S IgG für S. abortus equi 28,2 EU/100 mg, für E. Coli 34,2 EU/100 mg und für Pseudomonas aeruginosa 52,7 EU/100 mg.
      b) Bezogen auf einen Endotoxinüberschuß von 100 EU/dl beträgt die Inaktivierungskapazität:
         I.) bei Trf.-0̸ und 7S IgG für S. abortus equi 28,6 EU/ 100 mg, für E. Coli 30,7 EU/100 mg und für Pseudomonas aeruginosa 39,1 EU/100 mg.
         II.) bei Trf.-Fe(A) und 7S IgG für S. abortus equi 149,7 EU/100 mg, für E. Coli 237.7 EU/100 mg und für Pseudomonas aeruginosa 210,5 EU/100 mg.
         III,) bei Trf.-Fe(B) und 7S IgG für S. abortus equi 257,5 EU/100 mg, für E. Coli 358,4 EU/100 mg und für Pseudomonas aeruginosa 481,2 EU/100 mg.
   3.Transferrin und IgG/A/M (12% IgM)
      Bei Zusatz von Transferrin zu einer IgG -Präparation, die mit 12 % IgM angereichert ist (IgG/A/M), wird eine weitere Steigerung der Inaktivierungskapazität erreicht. Die Abnahme der Endotoxinaktivität beträgt bei Inkubation von Endotoxin mit der Kombination aus IgG/A/M (12% IgM ) ( 312 mg/dl ) und Transferrin, dessen Konzentration ebenfalls 312 mg/dl beträgt bei Verwendung von Transferrin (Trf.-Fe(A)), welches einen Eisengehalt von 5 µg pro Gramm Transferrin hat, je nach Art des Endotoxins 75.2 % bis 78.7%.
      Bei einem Eisengehalt des Transferrins (Trf.-Fe(B)) von 190 µg pro Gramm Transferrin und IgG/A/M, wird die Aktivität, je nach Endotoxin um 92% bis 96,5% herabgesetzt.
      a) Bezogen auf einen Endotoxinüberschuß von 10 EU/dl beträgt die Inaktivierungskapazität:
         I.) bei Trf.-Fe(A) und IgG/A/M für S. abortus equi 7,1 EU/100 mg, für E. Coli 11.1 EU/100 mg und für Pseudomonas aeruginosa 11.3 EU/100 mg.
         II.) bei Trf.-Fe(B) und IgG/A/M für S. abortus equi 88.5 EU/100 mg, für E. Coli 74,8 EU/100 mg und für Pseudomonas aeruginosa 88,9 EU/100 mg.
      b) Für einen Endotoxinüberschuß von 100 EU/dl beträgt die Inaktivierungskapazität:
         I.) bei Trf.-Fe(A) und IgG/A/M für S. abortus equi 99.3 EU/100 mg, für E. Coli 114.2 EU/100 mg und für Pseudomonas aeruginosa 112.5 EU/100 mg.
         II.) bei Trf.-Fe(B) und IgG/A/M für S. abortus equi 367.1 EU/100 mg, für E. Coli 782.2 EU/100 mg und für Pseudomonas aeruginosa 1173,5 EU/100 mg.
B) EINFLUSS AUF DIE FREISETZUNG DER MEDIATOREN IL-1 UND IL-6
   Als zusätzlicher Parameter für den Einfluß der untersuchten Verbindungen auf die biologische Aktivität des Endotoxins wurde die Freisetzung von IL-1 und IL-6 aus Monocyten herangezogen. Hierzu wurde Endotoxin zunächst mit Transferrin bzw. mit der Kombination von Transferrin und Immunglobulin inkubiert. Anschließend wurde der Überstand mit Monocyten inkubiert und die Konzentration der aus den Monocyten freigesetzten Mediatoren Interleukin-1 und Interleukin-6 im Überstand bestimmt. Es wurde Transferrin(Trf.Fe(A)) verwendet, welches einen Eisengehalt von 5 µg pro Gramm Transferrin hat, sowie Transferrin ( Trf.-Fe(B)) mit einem Eisengehalt von 190 µg pro Gramm Transferrin.
   Folgende Immunglobuline wurden verwendet: 7 S IgG ( Intraglobin^{R}, Sandoglobin^{R}) sowie IgG (IgG/A/M), angereichert mit 12% IgM ( Pentaglobin^{R} )
   Von folgenden Bakterienstämmen wurden Endotoxine verwendet:
   Salmonella abortus equi ( NOVOPYREXAL^{R} ), sowie der FDA Endotoxinstandard EC-5 von E. coli O113:H10:K0.
   Transferrin, Immunglobulin sowie Transferrin in Kombination mit Immunglobulin wurden mit Endotoxin 60 Minuten bei 37 °C inkubiert. Als Kontrolle wurde Human-Albumin verwendet. Die Konzentration des jeweiligen Proteins im Ansatz betrug: 625 mg/dl, 312 mg/dl und 156 mg/dl. Die Inkubation wurde mit Endotoxinkonzentrationen von 50 EU/dl bis 500 EU/dl durchgeführt.
   Nach der Inkubation wurde die jeweilige Endotoxinlösung mit mononukleären Zellen von gesunden Spendern inkubiert ( 16 Std. bei 37°C ). Anschließend wurde die Konzentration der freigesetzten Mediatoren IL-1 und IL-6 bestimmt. Die Bestimmung von IL-1 erfolgte mit Hilfe des Fibroblasten-Proliferationstest ( Loppnow H; Flad HD, Ulmer AJ et al (1989) "Detection of Interleukin 1 with Human Dermal Fibroblasts. Immunbiol. 179, 283 -291 ), unter Verwendung einer EL4-6.1 Thymomzell-Linie. Die Konzentration von IL-6 wurde ebenfalls über einen Proliferationstest unter Verwendung eines IL-6 abhängigen murinen Hybridoms ( 7TD1 ) bestimmt( Van Damme J, Cayphas S. et al. (1987) Eur. J. Biochem. 168: 543 -550: sowie Van Oers MHJ, Van der Heyden A. und Aarden LA (1988); Clin. exp. Immunol. 71: 314 -419 ).
   Es zeigte sich, daß die Inkubation von Endotoxin mit Transferrin zu einer starken Herabsetzung der Freisetzung von Mediatoren aus den mononukleären Zellen durch das Endotoxin führt. Dieser Effekt ist vom Eisengehalt des Transferrins abhängig, und zwar derart, daß er durch Erhöhung des Eisengehaltes verbesssert werden kann. Durch die Kombination von Transferrin mit Immunglobulinen kann der hemmende Effekt des Transferrins auf die Freisetzung von Mediatoren noch verstärkt werden, wobei auch in der Kombination mit Immunglobulinen eine bessere Wirkung bei höherem Eisengehalt des Transferrins erreicht wird.
   Ergebnisse: ( Abb. 3)
   I.Transferrin
      a) Bei Inkubation von 500 EU/dl Endotoxin mit Trf.Fe(A) betrug die mittlere Freisetzung von IL-1 bei S. abortus equi 211 U/ml und bei E. Coli 126 U/ml. Die Freisetzung von IL-6 betrug bei S. abortus equi 761 U/ml und bei E. Coli 1065 U/ml.
      b) Nach Inkubation von Endotoxin ( 500 EU/dl ) mit Trf.-Fe(B) betrug die mittlere Freisetzung von IL-1 bei S.abortus equi 81 U/ml und bei E. Coli 35 U/ml. Die mittlere Freisetzung von IL-6 betrug bei S. abortus 481 U/ml und bei E. Coli 415 U/ml.
   II.Transferrin und 7S IgG
      a) Bei Inkubation von Endotoxin (500 EU/dl) mit einer Kombination von Transferrin und einer 7S IgG -Präparation führte die Verwendung von Trf.-Fe(A) bei S. abortus equi zu einer IL-1-Freisetzung von 85 U/ml und bei E. Coli von 34 U/ml. Die IL-6-Menge, die freigesetzt wird, betrug bei S. abortus 561 U/ml und bei E.Coli 443 U/ml.
      b) Bei Verwendung von Trf.-Fe(B), betrug die mittlere Freisetzung von IL-1 bei S. abortus equi 37 U/ml und bei E. Coli von 16 U/ml. Die IL-6-Menge, die nach der Inkubation freigesetzt wird, betrug bei S. abortus 297 U/ml und bei E.Coli 271 U/ml.
   III.Transferrin und IgG/A/M (12% IgM)
      Der Zusatz von Transferrin zu einer 7S IgG -Präparation, die mit 12 % IgM angereichert ist( IgG/A/M), führte bei einer Immunglobulingesamtkonzentration von 312 mg% und einer Transferrinkonzentration, die ebenfalls 312 mg% betrug, nach Inkubation mit 500 EU/dl Endotoxin:
      a) bei Verwendung von Trf.-Fe(A) zu einer Freisetzung von IL-1, von 173 U/ml bei S. abortus equi und von 127 U/ml bei E. Coli. Die IL-6-Menge, die freigesetzt wurde, betrug bei S. abortus equi 678 U/ml und bei E.Coli 1065 U/ml.
      b) Bei Kombination von Trf.-Fe(B) mit IgG/A/M(12%) betrug die mittlere Freisetzung von IL-1 bei S. abortus equi 19 U/ml und bei E. Coli 4.5 U/ml. Die mittlere Freisetzung von IL-6 betrug unter diesen Bedingungen bei S. abortus equi 204 U/ml und bei E. Coli 118 U/ml.
   IV ALBUMIN
      Nach Inkubation von Endotoxin( 500 EU/dl ) mit Albumin, welches zum Vergleich verwendet wird, beträgt, die mittlere Freisetzung von IL-1 bei Verwendung des Endotoxin von S. abortus equi 2975 U/ml und bei E. Coli 2676 U/ml. Die Freisetzung von IL-6 beträgt bei S. abortus equi 30̸18 U/ml und bei E. Coli 6233 U/ml.
C) Einfluß auf die Endotoxinaktivität im Plasma
   Humanplasma von gesundenen Spendern wurde mit Transferrin versetzt derart, daß der natürlich vorhandene Transferringehalt im Plasma um 312 mg % erhöht wurde. Den Kontrollproben wurde die gleiche Menge Albumin zugesetzt. Zu dem mit Transferrin bzw. Albumin versetzten Plasma wurde dann Endotoxin ( Pseudomonas-Endotoxin, Salmonellen-Endotoxin ) zugesetzt, sodaß im Ansatz eine Endotoxinaktivität von 800 EU/dl und 300 EU/dl im Plasma erreicht wurde. Der Ansatz wurde dann 60 Minuten bei 37°C inkubiert. Anschließend wurde die verbliebene Endotoxinaktivität bestimmt.
   Es zeigte sich, daß die Fähigkeit des Plasmas zur Inaktivierung von Endotoxin gegenüber den Proben, denen nur Albumin zugesetzt wurde, durch den Transferrinzusatz signifikant gesteigert wird.
   a) bei Zusatz von Transferrin ( Trf.-Fe(B) ) betrug die Steigerung der Endotoxininaktivierung: 73.8% bei 800 EU/dl Endotoxin und bei 300 EU/dl Endotoxin : 56.5%
   b) bei Zusatz von Trf. -Fe(A) wurde die Endotoxininaktivierung im Plasma bei einer Endotoxinkonzentration von 800 EU/dl um 66.6% und bei 300 EU/dl Endotoxin um 42.8 % gesteigert.

### Tierexperimentelle Untersuchungen

Die tierexperimentellen Untersuchungen zur Abklärung der Frage, welche Wirkung Transferrin sowie die Kombination von Transferrin mit Immunglobulinen auf die Endotoxinaktivität haben, wurden an folgendem Tiermodell durchgeführt:

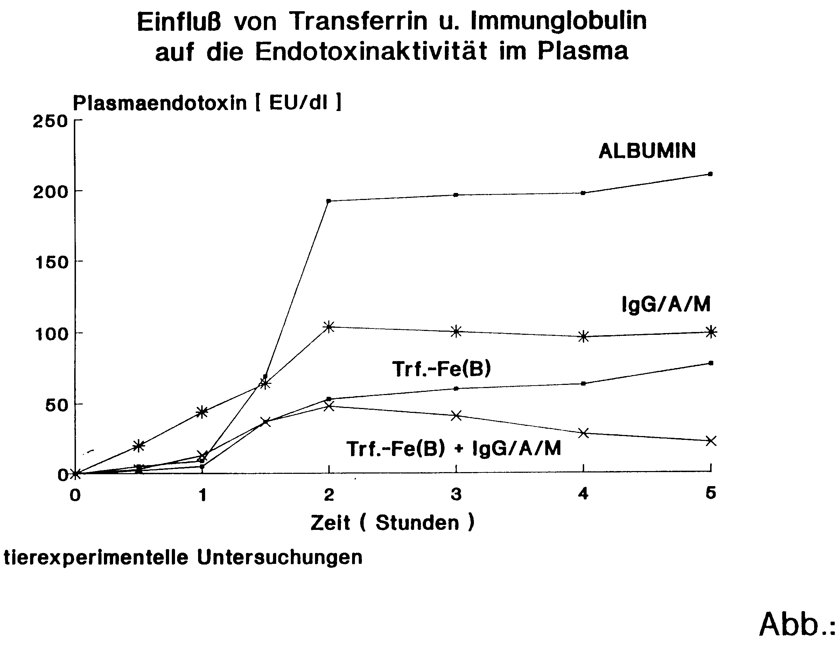

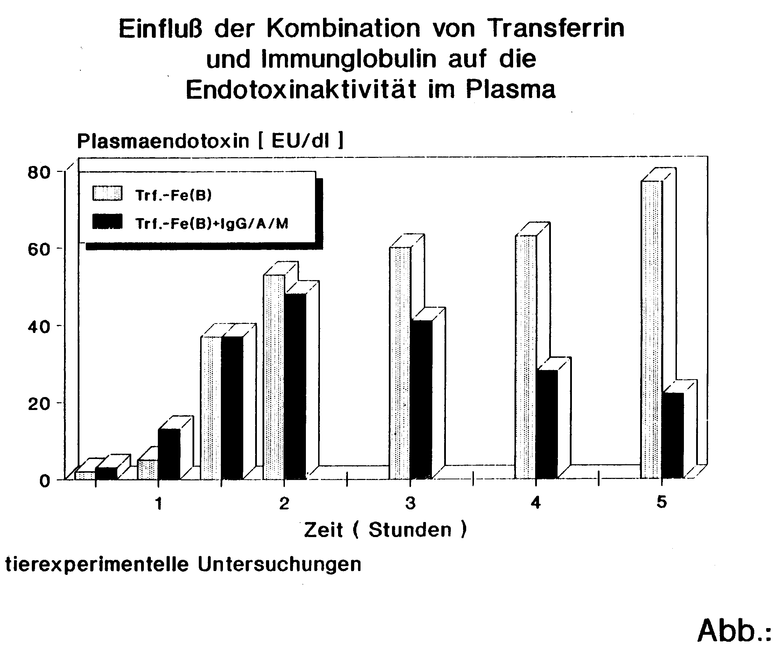

Narkotisierten Wistar-Ratten (250 - 300 g) wurde zur Blutabnahme zunächst ein Katheter in die rechte Halsvene (Vena Jugularis ) eingelegt. Anschließend wurde eine definierte Zahl verschiedener Bakterien ( E. Coli. Klebsiella species und Pseudomonas aeruginosa; in einer Konzentration von jeweils 0.75 x 10 ⁵CFU/ml ) in die Bauchhöhle appliziert. 30 Minuten nach Gabe der Bakterien in die Bauchhöhle wurde über den Venenkatheter mit einem Perfusor langsam das zu prüfende Präparat ( Transferrin, sowie Transferrin und Immunglobulin ) in einer Dosierung von 250 mg / Kg Körpergewicht intravenös verabreicht. Die Kontrolltiere erhielten Albumin in entsprechender Dosierung.
Zur Erzeugung eines starken Endotoxineinstroms in das Blut wurde eine Stunde nach der Bakteriengabe ein bakterizides Antibiotikum (IMIPENEM = Zienam^{R}) intravenös appliziert.
Die Blutabnahmen zur Bestimmung der Endotoxinaktivität sowie der Bakterienzahl im Blut erfolgten vor, sowie - in 30 minütigem Abstand - nach Bakteriengabe über insgesamt 5 Stunden.
Folgende Präparationen wurden i.v. verabreicht:
7S IgG (Sandoglobin^{R}): IgM(12%)-angereichertes 7S IgG (Pentaglobin^{R}), Transferrin ( Trf.-Fe(A)) mit einem Eisengehalt von 25 µg/dl - bezogen auf eine 5% Transferrinlösung - und Transferrin (Trf.-Fe(B)) mit einem Eisengehalt von 950 µg/gl in einer 5% Transferrin-Lösung: sowie die Kombination von Transferrin (TRF.-Fe(A) und TRF.-Fe(B)) mit IgM(12%)-angereichertem Immunglobulin und Transferrin ( Trf.-Fe(A) u. Trf.-Fe(B)) mit 7 S IgG ( Sandoglobin^{R} ).
Ergebnisse: ( Abb. 4 )
a) Bei Gabe von Albumin steigt die Endotoxinaktivität im Plasma nach Applikation des Antibiotikums sehr stark an. Bereits eine Stunde nach Gabe des Antibiotikums wird eine mittlere Endotoxinaktivität von 192 EU/dl erreicht. Vier Stunden nach Gabe des Antibiotikums beträgt die Endotoxinaktivität im Plasma 210 EU/dl.
b) Die i.v.-Gabe von IgM (12%)-angereichertem 7S IgG führt zu einer Senkung der Endotoxinaktivität im Plasma, die eine Stunde nach Gabe des Antibiotikums, im Vergleich zu der Albumin-Kontrollgruppe. ca. 46 % und vier Stunden später 53% beträgt.
c) Bei i.v.-Gabe von Transferrin ( Trf.-Fe(A) ) wurde die initiale Zunahme der Endotoxinaktivität im Plasma - eine Stunde nach Gabe des Antibiotikums - im Vergleich zur Albumin-Kontrollgruppe um ca 36 % reduziert.
d) Bei i.v.-Gabe von Transferrin ( Trf.-Fe(B) ) beträgt die Reduzierung der initialen Zunahme der Endotoxinaktivität im Plasma - eine Stunde nach Gabe des Antibiotikums - im Vergleich zur Albuminkontrollgruppe ca. 72.5 %. Vier Stunden nach Gabe des Antibiotikums ist die Endotoxinaktivität bei der Gruppe, die Trf.-Fe(B) erhalten hat, noch um ca. 63 % niedriger, als bei der Albumin-Gruppe.
e) Bei i.v.-Gabe von Transferrin ( Trf.-Fe(B) ) in Kombination mit einem Immunglobulin, welches mit 12% IgM angereichert ist, ist die initiale Zunahme der Endotoxinaktivität im Plasma - eine Stunde nach Gabe des Antibiotikums - im Vergleich zur Albuminkontrollgruppe ca. 75 % niedriger. Vier Stunden nach Gabe des Antibiotikums ist die Endotoxinaktivität bei der Gruppe, welche diese Kombination erhalten hat, ca. 90 % niedriger, als bei der Albumin-Gruppe.

### Zusammenfassende Beurteilung der Ergebnisse:

1. Transferrin ist geeignet, bei intavenöser Gabe, konzentrationsabhängig die biologische Aktivität des Endotoxins im Blut herabzusetzen.
2. Das Ausmaß, in dem die toxische Wirkung des Endotoxins durch das Transferrin herabgesetzt wird, nimmt mit der Eisenbeladung des Transferrins zu.
   Der Eisengehalt des Transferrins sollte hierbei mindestens 0.4 µg pro Gramm Transferrin betragen, vorzugsweise jedoch höher als 100 µg pro Gramm Transferrin sein.
3. Transferrin, welches frei von Eisen ist, hat eine schwache Wirkung auf die Endotoxinaktivität.
4. Durch die Kombination von Transferrin mit anderen Plasmaproteinen, insbesondere mit Immunglobulinen der IgG- und IgM- Fraktion wird eine signifikant höhere Wirkung auf die biologische Aktivität des Endotoxins erreicht, als sie bei den einzelnen Proteinfraktionen zu finden ist.
   Der Synergismus in der Wirkung auf die Endotoxinaktivität führt bei Kombination von Transferrin und Immunglobulinen zu einer Potenzierung des inaktivierenden Effektes beider Proteine.
5. Aufgrund des Synergismus zeichnet sich die Kombination von Transferrin mit Immunglobulinen durch eine wesentliche Erhöhung der Inaktivierungskapazität für Endotoxin aus.
6. Eine Herabsetzung der Endotoxinaktivität ist zwar auch bei Kombination von eisenfreiem Transferrin mit Immunglobulin möglich, doch um eine größere Inaktivierungskapazität zu erreichen, erweist es sich als günstiger, wenn der Eisengehalt des Transferrins mindestens 0,4 µg pro Gramm Transferrin beträgt. Vorzugsweise sollte der Eisengehalt jedoch höher als 100 µg pro Gramm Transferrin sein.
7. Aufgrund seiner Fähigkeit, die biologische Aktivität des Endotoxins im Blut herabzusetzen, eignet sich besonders die intravenöse Gabe des eisenbeladenen Transferrins für die Bekämpfung der toxischen Wirkung von Endotoxin, welches in die Blutbahn übergetreten ist.
8. Aufgrund der größeren Inaktivierungskapazität ist die Kombination von Transferrin mit Immunglobulinen für therapeutische Maßnahmen zur Bekämpfung der toxischen Wirkung von Endotoxin, besonders bei allen krankhaften Veränderungen geeignet, bei denen es zu einem stärkeren und länger andauernden Endotoxinübertritt in die Blutbahn kommt.
9. Bei rechtzeitigem therapeutischen Einsatz von Transferrin bzw. der Kombination von Transferrin mit Immunglobulinen ist ein günstiger Effekt auf den klinischen Verlauf bei Endotoxinämien jeglicher Genese zu erwarten.

### Die Erfindung wird durch folgende Beispiele erläutert.

### BEISPIEL 1

Bei Inkubation von 200 EU/dl Endotoxin von S. abortus equi mit einem Immunglobulin, welches mit 12% IgM-angereichert ist ( IgG/A/M ), in einer Konzentration von 312 mg/dl, wird die Endotoxinaktivität in vitro um ca 42% herabgesetzt. Bei der gleichen Konzentration ( 312 mg/dl) von Transferrin ( Trf. -Fe(A) ) mit einem Eisengehalt von 25 µg/dl - bezogen auf eine 5% Lösung - wird die Endotoxinaktivität in vitro um 73 % (S. abortus equi) bzw. 76% ( Pseudomonas aeruginosa ) und durch Transferrin(Trf.-Fe(B)) mit einem Eisengehalt von 950̸ µg/dl - bezogen auf eine 5% Transferrin-Lösung - um maximal 85 % ( S.abortus equi) bzw. 91% ( Pseudom. aeruginosa ) herabgesetzt.

Durch die Kombination von Trf.-Fe(B) mit dem Immunglobulin (IgG/A/M) wird bei niedriger Endotoxinaktivität, d.h. bis 200 EU/dl, im Vergleich zu der Inaktivierung bei alleiniger Gabe von Trf.-Fe(B), keine signifikante Verbesserung der Ianktivierung erreicht. Dagegen zeichnet sich die Kombination von Trf. -Fe(B) mit Immunglobulin dadurch aus, daß sie auch bei höherer Endotoxinaktivität - d.h. über 300 EU/dl Endotoxin - signifikant mehr Endotoxin inaktiviert, als es die beiden Proteine allein vermögen. Während die Kombination von Trf.-Fe(B) mit IgG/A/M(12%) bei Inkubation mit 750 EU/dl Endotoxin von S.abortus equi 92 % des Endotoxins inaktivieren kann, werden durch Trf. -Fe(B) nur maximal 85 % der Aktivität neutralisiert.
Die Unterschiede zwischen Trf.-Fe(B) und der Kombination mit Immunglobulin werden durch die Angabe der Inaktivierungskapazität ( Abb.: 2) wesentlich deutlicher, als durch die Angabe der mittleren prozentualen Inaktivierung. Beispielsweise ist diese Kapazität - bezogen auf einen Endotoxinüberschuß von 100 EU/dl - für die Inaktivierung des Endotoxins von Pseud. aeruginosa bei Verwendung der Kombination von Trf.-Fe(B) und Immunglobulin (IgGA/M) um einen Faktor von 2,7 größer, als die Inaktivierungskapazität von Trf. -Fe(B) allein und um einen Faktor von 17,3 größer als die Inaktivierungskapazität der Immunglobulinpräparation IgG/A/M (12%) .
Die Steigerung der Inaktivierungskapazität durch die Kombination von Transferrin mit Immunglobulin eröffnet die Möglichkeit, daß durch eine therapeutische Zufuhr dieser Kombination auch stärkere und länger andauernde Endotoxineinschwemmungen in die Blutbahn, wie sie beispielsweise auch bei der diffusen Peritonitis auftreten können, aufgefangen und behandelt werden können.

### BEISPIEL 2 (Abb. 4 u. 5 )

Entsprechend den Bedingungen, wie sie bei der Therapie von gramnegativen Infektionen mit Antibiotika auftreten können, wurde im Tierversuch ein Endotoxinanstieg im Blut dadurch induziert, daß zunächst Bakterien in die Bauchhöhle gegeben werden und durch die anschließende intravenöse Gabe eines bakteriziden Antibiotikums ein rascher Zerfall dieser Bakterien ausgelöst wird. Vom Zeitpunkt der Gabe des Antibiotikums an kann im Blut ein starker Anstieg der Endotoxinaktivität gemessen werden. Wird vorher ein Immunglobulin oder eisenhaltiges Transferrin oder die Kombination von Transferrin(Trf.-Fe(B)) mit Immunglobulin intravenös verabreicht, dann wird der Anstieg des Endotoxinspiegels im Vergleich zu einer Kontrollgruppe, die stattdessen Albumin i.v. erhielt, je nach verwendetem Plasma-Protein, mehr oder minder stark herabgesetzt. Beispielsweise wird die mittlere maximale Endotoxinaktivität, die bei der Kontrollgruppe bei Versuchsende 210 EU/dl beträgt, durch die Gabe von Transferrin auf 77 EU/dl gesenkt. Bei Gabe der Kombination von Transferrin(Trf.-Fe(B) und Immunglobulin (IgG/A/M) beträgt die Endotoxinaktivität im Plasma nur noch 22 EU/dl.

Durch die Steigerung der Inaktivierungskapazität bei Kombination von Transferrin mit Immunglobulin erhält man damit die Möglichkeit, auch bei einem längeren Endotoxineinstrom einen stärkeren Anstieg der Endotoxinaktivität im Plasma zu verhindern. Die alleinige Gabe von eisengebundenem Transferrin bewirkt initial eine ähnlich starke Reduzierung der Endotoxinaktivität wie die Kombination von Transferrin mit Immunglobulin ( Abb.: 5 ). Ohne Erhöhung der Dosis reicht jedoch die Inaktivierungskapazität von Transferrin, allein, bei einem fortgesetzten Endotoxinübertritt in das Blut nach einer gewissen Zeit nicht mehr aus, wodurch es wieder zu einem Ansteigen der Endotoxinaktivität im Plasma kommen kann.

### BEISPIEL 3

Bei einer Patientin, die das klinische Bild einer schweren abdominalen Sepsis mit Organversagen entwickelt hatte, konnte eine erhöhte Endotoxinaktivität im Plasma nachgewiesen werden. Die Konzentration des Transferrins im Plasma betrug 98 mg/dl ( Normalbereich: 200 - 400 mg/dl ). Bei Inkubation dieses Plasmas mit einer Immunglobulin-Präparation, die mit IgM-angereichert ist, ( 30 Minuten, 37 °C) - Immunglobulinkonzentration im Ansatz: 312 mg/dl - konnte eine Abnahme der Endotoxinaktivität um 17% erreicht werden. Bei Inkubation mit Transferrin(Trf.-Fe(A)), in einer Konzentration von 312 mg/dl, werden 29% des Endotoxins inaktiviert. Durch Kombination von Trf.-Fe(A) mit dem Immunglobulin in einer Konzentration von jeweils 312 mg/dl wird die Endotoxininaktivierung im Plasma der Patientin auf 39 % gesteigert.
Bei Zugabe der gleichen Menge von Transferrin(Trf.-Fe(B) mit einem höheren Eisengehalt, in entsprechender Konzentration, zum Patientenplasma werden 45 % der Endotoxine inaktiviert. Wird das Transferrin (Trf.-Fe(B)) mit dem IgM-angereicherten Immunglobulinpräparat(IgG/A/M) kombiniert, dann kann sogar 53 % des Endotoxins im Plasma der Patientin, d.h. 36 % mehr als mit dem reinen Immunglobulinpräparat inaktiviert werden. Die starke Senkung der Endotoxinaktivität durch Transferrin und die noch stärkere Senkung der Aktivität durch die Kombination von Transferrin mit Immunglobulinen geben bei intravenöser Applikation die Möglichkeit, die Prognose des Patienten bei Endotoxinämien jeglicher Genese entscheidend zu verbessern.

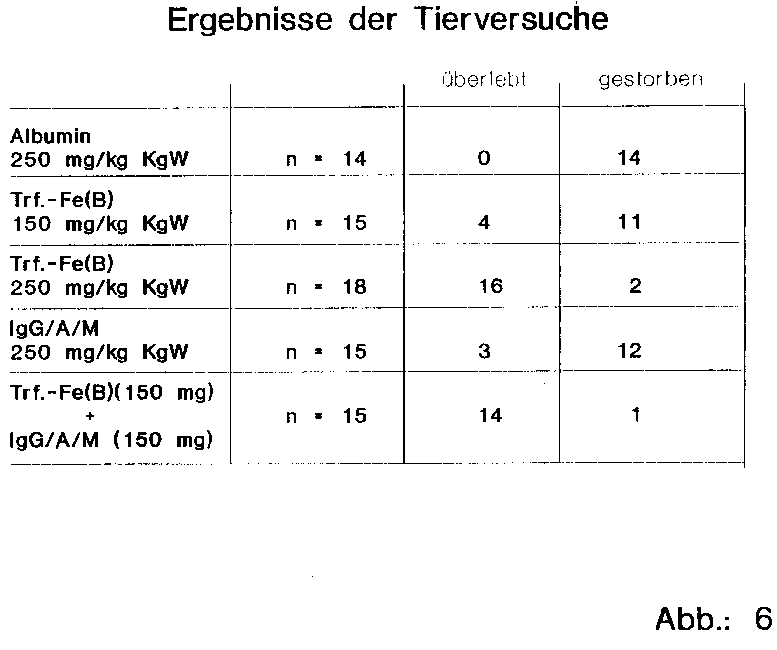

### BEISPIEL 4 ( Abb.6 )

Zur Prüfung der Frage, welchen Einfluß die Kombination von Transferrin mit einem Immunglobulin auf die Letalität im Tierversuch hat, wurde Wistar-Ratten eine definierte Zahl von Coli-Bakterien 9 x 10⁹ CFU/ml intraperitoneal verabreicht. Zur Erzeugung einer ausgeprägten Endotoxinämie wurde 30 Minuten später ein bakterizides Antibiotikum [ IMIPENEM : 28 mg/kg Körpergewicht ] i.v. appliziert. Um eine möglichst radikale Sanierung der bakteriellen Infektion zu erreichen, wurde das Antibiotikum in einer Dosierung von 14 mg/kg in 4-stündigem Abstand über insgesamt 3 Tage weiter verabreicht.
Einer Gruppe( n = 18 ) der Tiere wurde 15 Minuten vor Bakteriengabe eisenreiches Transferrin ( Trf.-Fe(B) ) in einer Dosierug von 250 mg/kg intravenös verabreicht. Eine weitere Gruppe(n=15) erhielt Transferrin( Trf.-Fe(B) in einer Dosierung von 150 mg/kg i.v. Eine andere Gruppe( n=15) erhielt vor Gabe der Bakterien eine Immunglobulinpräparation mit einem IgM-Gehalt von 12% IgM ( IgG/A/M ) in einer Dosierung von 250 mg/kg intravenös. 15 Tiere erhielten eine Kombination von Transferrin (Trf.-Fe(B)) und der mit 12% IgM-angereicherten polyvalenten IgG-Präparation (IgG/A/M) in einer Dosierung von jeweils 150 mg/kg. Die Kontrollgruppe erhielt vor Bakteriengabe nur Albumin i.v , in einer Dosierung von 250 mg/kg.
Während sämtliche Tiere der Albumingruppe und 80% der Tiere der Immunglobulin-Gruppe (IgG/A/M) spätestens am 5. Tag nach Bakteriengabe verstorben waren, überlebten 89% der Tiere in der Gruppe, welche auschließlich 250 mg/dl Transferrin (Trf.-Fe(B)) erhalten hatte. Im Gegensatz dazu verstarben 74% der Tiere, die Transferrin ( Trf.-Fe(B) ) in einer Dosierung von 150 mg/kg bekommen hatten. Von den Tieren, welche die Kombination von Transferrin (Trf.-Fe(B)) und Immunglobulin(IgG/A/M) erhalten hatten, überlebten 94%.

## Patentansprüche

1. Verwendung eine Mittels bestehend aus Transferrin zur Herstellung eines Arzneimittels zur Bekämpfung der toxischen Wirkung des Endotoxins bei Endotoxinämien, dadurch gekennzeichnet, daß das Transferrin Eisen gebunden hat.

2. Verwendung nach Anspruch 1, dadurch gekennzeichnet, daß das Transferrin ein oder zwei Eisen-Ionen gebunden hat.

3. Verwendung nach Anspruch 1 oder Anspruch 2, gekennzeichnet durch die Kombination von Transferrin mit Immunglobulinen.

4. Verwendung nach Anspruch 1 oder Anspruch 2, gekennzeichnet durch die Anreicherung von Transferrin in einer Serumkonserve.

## Claims

1. Use of substance consisting of transferrin for the manufacture of a medicament to combat the toxic effects of endotoxines in endotoximia, characterized in that the transferrin has bound iron.

2. Use according to claim 1, characterized in that the transferrin has bound one or two ions of iron.

3. Use according to claim 1 or claim 2, characterized by the combination of transferrin with immunglobulin.

4. Use according to claim 1 or claim 2, characterized by the enrichment of transferrin in a stored serum.

## Revendications

1. Utilisation d'un agent se composant de transferrine pour la production d'un médicament destiné a combattre les effets toxiques de l'endotoxine en cas d'endotoxinémies, caractérisé par le fait que la transferrine a lié du fer.

2. Utilisation selon la revendication 1, caractérisée par la transferrine a lié un ou deux ions de fer.

3. Utilisation selon la revendication 1 ou 2, caractérisée par la combinaison de transferrine avec des immun-globulines.

4. Utilisation selon la revendication 1 ou 2, caractérisée par l'enrichissement de transferrine dans un sérum conservé.
